# EUROPEAN PATENT APPLICATION

(11) **EP 4 843 508 A1**
(43) Date of publication of application: **25.11.2026**
(21) Application number: 24917983.9
(22) Date of filing: 09.07.2024
(51) Int. Cl.: A61B 8/12, A61B 8/00, A61M 25/09, A61M 25/01, A61M 25/00

(54) **INTERVENTIONAL TREATMENT SYSTEM AND ULTRASONIC CATHETER FOR INTERVENTIONAL TREATMENT**

(30) Priority: 18.01.2024 CN 202410074720
(71) Applicant: Acoustic Life Science Co., Ltd., Shanghai 201203 (CN)
(72) Inventor: LI, Mingkan, Shanghai 201203 (CN); YAN, Zhibing, Shanghai 201203 (CN); LI, Xiang, Shanghai 201203 (CN)
(74) Representative: Gille Hrabal Partnerschaftsgesellschaft mbB Patentanwälte
(86) International application number: PCT/CN2024/104465
(87) International publication number: WO 2025/152370

(57) **Abstract**

The present disclosure relates to the field of interventional treatment technology, and in particular, to an interventional treatment system and an ultrasound catheter for interventional treatment thereof. The ultrasound catheter for interventional treatment comprises: a dual-lumen microcatheter, comprising a first guide lumen and a first core lumen; an acoustically transparent section, comprising a second core lumen and a second guide lumen, the second guide lumen being provided with a guidewire outlet, and the first core lumen and the second core lumen both extending in a pushing direction and being arranged in parallel to each other; a core, provided with an ultrasound transducer arranged at the distal end thereof, the core being arranged within the second core lumen and the first core lumen, and the ultrasound transducer being arranged within the second core lumen; and a rapid exchange section, connected to the distal end of the acoustically transparent section, the distal end of the rapid exchange section allowing the entrance of a directional guidewire, and the proximal end of the rapid exchange section allowing the exit of the directional guidewire. The apparatus integrates the functions of diagnostic intravascular ultrasound imaging and guidewire intervention, and can direct guidewire puncture in real time under IVUS guidance and create a channel into the vascular true lumen distal to the occlusion section, thus improving the success rate of the operation.

## Description

The present application claims priority to Chinese Patent Application No. 202410074720.6 filed to China National Intellectual Property Administration on Jan. 18, 2024 and entitled "INTERVENTIONAL TREATMENT SYSTEM AND ULTRASOUND CATHETER THEREOF FOR INTERVENTIONAL TREATMENT", which is incorporated herein by reference in its entirety.

### TECHNICAL FIELD

The present disclosure relates to the field of interventional treatment technology, and particularly, to an ultrasound catheter for interventional treatment. Furthermore, the present disclosure relates to an interventional treatment system comprising the ultrasound catheter described above for interventional treatment.

### BACKGROUND

In the prior art, conventional catheters with ultrasound capabilities are of single-lumen structure designed exclusively for ultrasound transducer imaging. They possess limited functionality and cannot directly achieve therapeutic effects. For an instrument for interventional treatment integrated with ultrasound functionality, various interventional treatment devices should be connected in series on the same first guidewire to reach the target location. The great overall radial size limits its application scenes and leads to inconvenience in achieving synchronized operation between ultrasonographic devices and the interventional treatment devices. In particular, once an interventional treatment device enters another branch, the ultrasonographic devices can no longer follow for real-time image guidance. Especially for a surgical operation with complicated procedures, operating multiple devices arranged serially on a single first guidewire requires a strictly and thoroughly designed sequence; otherwise, repeated retraction and repositioning of the devices within the body will be necessary, making the procedures complicated, and resulting in poor ultrasonographic quality, difficulties in maintaining continuous ultrasound-directed visualization, and prolonged and challenging surgeries. For example, in lesions where the blood vessel is completely occluded, it may be difficult to distinguish the direction of the vascular intima and the occluded section without real-time image guidance, thus failing to effectively maneuver a guidewire across the vascular intima to re-enter the true lumen or find the entrance to the vascular occlusion.

In summary, how to provide the catheter with functions of both image guidance and interventional treatment, reduce the operation time and difficulty, and improve the success rate of the operation is an urgent problem to be solved by those skilled in the art at present.

### SUMMARY

In view of this, the present disclosure is intended to provide an ultrasound catheter for interventional treatment, which integrates the functions of diagnostic intravascular ultrasound (IVUS) imaging and guidewire treatment, and can direct guidewire puncture in real time under IVUS guidance and create a channel into the vascular true lumen distal to the occluded section, thereby effectively reducing the operation time and difficulty, and improving the success rate of the operation. The present disclosure is also intended to provide an interventional treatment system comprising the ultrasound catheter for interventional treatment described above.

To achieve the aforementioned objectives, the present disclosure provides the following technical solution:
Provided is an ultrasound catheter for interventional treatment, comprising: a dual-lumen microcatheter, comprising a first guide lumen and a first core lumen; an acoustically transparent section, comprising a second core lumen in communication with the distal end of the first core lumen and a second guide lumen in communication with the distal end of the first guide lumen, the second guide lumen being provided with a guidewire outlet, and the second core lumen and the second guide lumen both extending distally and being arranged in parallel to each other; a core, provided with an ultrasound transducer arranged at the distal end thereof, the core being arranged within the second core lumen and the first core lumen, and the ultrasound transducer being arranged within the second core lumen; and a rapid exchange section, connected to the distal end of the acoustically transparent section, the distal end of the rapid exchange section being configured for allowing the entrance of a directional guidewire, and the proximal end of the rapid exchange section being configured for allowing the exit of the directional guidewire, wherein, the ultrasound catheter for interventional treatment further comprises a Y-shaped connection member and a connector; the Y-shaped connection member comprises a third core lumen in communication with the proximal end of the first core lumen and a third guide lumen in communication with the proximal end of the first guide lumen; the connector comprises an irrigation port and a core port, both in communication with the third core lumen.

In some embodiments, the ultrasound transducer is configured for selecting a target position, the ultrasound transducer is configured for acquiring an image in a radial direction of the second core lumen, and the ultrasound catheter can be rotated such that the guidewire outlet and the target position are arranged on the same side of the ultrasound transducer.

In some embodiments, the acoustically transparent section is made of a material that is readily transparent to sound; the cross-sectional profile of the acoustically transparent section is circular, or the cross-sectional profile of the acoustically transparent section transitions from circular to elliptical as the acoustically transparent section extends distally.

In some embodiments, the cross-sectional profile of the acoustically transparent section is elliptical; and the ratio of a minor axis to a major axis of the elliptical acoustically transparent section is 1:1.7 to 1:2.3, and/or the ratio of a minimum wall thickness to a maximum wall thickness of the elliptical acoustically transparent section is 1:4 to 1:6.

In some embodiments, the first core lumen and the second core lumen are both cylindrical cavities, and the core can move forwards or backwards relative to the first core lumen and the second core lumen; and the ultrasound transducer has an initial position substantially flush with the guidewire outlet, and the ultrasound transducer has a detection position when moving relative to the guidewire outlet.

In some embodiments, the rapid exchange section has a length L1, the distance from the distal end of the ultrasound catheter to the proximal end of the acoustically transparent section is (L1 + Lt), and the ratio of L1 to (L1 + Lt) is 1/5 to 1/7.

In some embodiments, the acoustically transparent section is provided with an arc-shaped profile at the guidewire outlet, and the radial dimension of the acoustically transparent section gradually tapers as the acoustically transparent section extends distally; and/or the rapid exchange section is provided with an arc-shaped hole for allowing the directional guidewire to pass through in a bent state.

In some embodiments, the dual-lumen microcatheter comprises an inner polymer layer, an intermediate reinforcement layer, and an outer resin layer in sequence from inside to outside; the inner polymer layer is made of a polymer material with a low coefficient of friction, and the inner polymer layer forms the first guide lumen and the first core lumen; the intermediate reinforcement layer is formed by braiding or winding around or sheathing the inner polymer layer; the outer resin layer is made of at least two materials with different hardnesses, and the hardness of the outer resin layer gradually decreases from the proximal end to the distal end; the inner polymer layer, the intermediate reinforcement layer, and the outer resin layer are formed into the ultrasound catheter by the co-extrusion technology or heat shrink tubing technology.

Provided is an interventional treatment system, comprising a number of guidewires and the ultrasound catheter for interventional treatment according to any one of the foregoing; the guidewires include an interventional guidewire extending out through the guidewire outlet and a directional guidewire passing through the rapid exchange section; the directional guidewire is configured for directing the ultrasound catheter to a desired position, and the interventional guidewire is configured for delivering to a target position under the imaging guidance of the ultrasound catheter; the guidewire can be switched between a status of extending out through the guidewire outlet and a status of passing through the rapid exchange section, such that the guidewire can be switched between the directional guidewire and the interventional guidewire.

In some embodiments, the interventional treatment system further comprises an interventional medical device, wherein the interventional medical device comprises at least one of a working guidewire, a balloon, a stent, a rotary milling device, and a suction device; the interventional medical device is configured for delivering to the target position along the guidewire, or the interventional medical device is configured for extending out through the guidewire outlet and delivering to the target position directly under the imaging guidance of the ultrasound catheter.

In some embodiments, the interventional treatment system is configured for interventional occlusion treatment; the guidewires further include an interventional treatment guidewire; the distal end of the interventional treatment guidewire is arranged at the distal end of an occluded vascular section, the proximal end of the interventional treatment guidewire is arranged at the proximal end of the occluded vascular section, and a middle section of the interventional treatment guidewire bypasses the occluded vascular section under the imaging direction of the ultrasound catheter through an interlayer between the intima and media of the occluded vascular section.

In summary, the interventional treatment system and the ultrasound catheter for interventional treatment provided herein integrate the functions of diagnostic intravascular ultrasound (IVUS) imaging and interventional treatment, and can establish an interventional treatment path or directly perform the treatment under the direction of IVUS. Due to the integrated structure of the ultrasound catheter, the accuracy of image direction can be better ensured, and the ultrasonographic effect is improved. Moreover, since the functions of directing to the target position and performing treatment on the target position are independent without sharing the directional path with other devices during the interventional treatment, the overall outer diameter can be reduced, and the range of application can be expanded. In addition, as the catheter allows the guidewire to be switched to the directional guidewire or the interventional guidewire at different stages, the ultrasound transducer of the catheter can provide a consistent image direction in various stages, thereby effectively reducing the operation time and difficulty, and improving the success rate of the operation.

### BRIEF DESCRIPTION OF THE DRAWINGS

To more clearly illustrate the embodiments of the present disclosure or the technical solutions in the prior art, the drawings needed in the description of the embodiments or the prior art are briefly described below. It is obvious that the drawings in the description below are only illustrative of the embodiments described in the present disclosure, and those of ordinary skill in the art can acquire other drawings according to the drawings without creative efforts.
FIG. 1 is a structural schematic view of the distal end of an ultrasound catheter for interventional treatment provided herein;
FIG. 2 is a structural schematic view of the proximal end of an ultrasound catheter for interventional treatment provided herein;
FIG. 3 is a schematic structural view of a dual-lumen microcatheter;
FIG. 4 is a schematic view of an end part of an acoustically transparent section;
FIG. 5 is a structural schematic view of an acoustically transparent section;
FIG. 6 is a structural schematic view of a rapid exchange section;
FIG. 7 is a schematic structural view of a Y-shaped connection member;
FIG. 8 is a structural schematic view of a connector;
FIG. 9 is a structural schematic view of a core;
FIG. 10 is a structural schematic view of a second guidewire and a first guidewire;
FIG. 11 is a structural schematic view of the dual-lumen ultrasound catheter entering an intima;
FIG. 12 is a structural schematic view of the dual-lumen ultrasound catheter entering a branch blood vessel of an occluded section;
FIG. 13 is another structural schematic view of the dual-lumen microcatheter;
FIG. 14 is a schematic view of the length of the rapid exchange section;
FIG. 15 is a schematic view of the length of the rapid exchange section;
FIG. 16 is a schematic structural view when the acoustically transparent section has an elliptical profile;
FIG. 17 is an ultrasonogram of the acoustically transparent section of FIG. 16;
FIG. 18 is a schematic structural view when the acoustically transparent section has a circular profile; and
FIG. 19 is an ultrasonogram of the acoustically transparent section of FIG. 18.

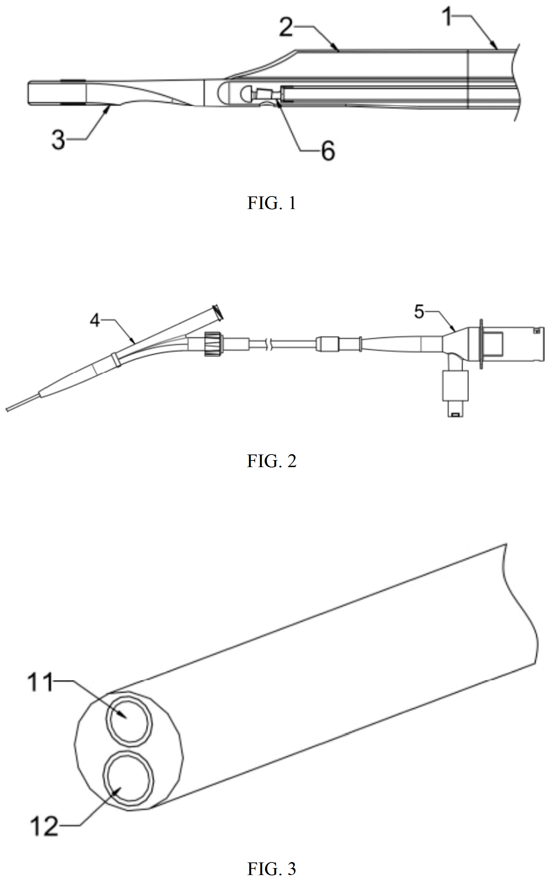

In FIGs. 1-19:
1 is a dual-lumen microcatheter; 11 is a first guide lumen; 12 is a first core lumen; 2 is an acoustically transparent section; 21 is a second core lumen; 22 is a second guide lumen; 23 is a liquid outlet; 24 is a guidewire outlet; 3 is a rapid exchange section; 31 is a distal outer tube; 32 is a TIP; 33 is a radiopaque marker ring; 4 is a Y-shaped connection member; 41 is a third guide lumen; 42 is a third core lumen; 5 is a connector; 51 is an irrigation port; 52 is a core port; 6 is a core; 61 is an ultrasound transducer; 62 is a transducer base; 63 is a torque spring; 64 is a core connector; 7 is a second guidewire; 8 is a first guidewire; 9 is a third guidewire; 02 is a circular profile; 03 is an RX rapid exchange section.

### DETAILED DESCRIPTION

The technical solutions in the embodiments of the present disclosure will be clearly and completely described below with reference to the drawings in the embodiments of the present disclosure, and it is obvious that the described embodiments are only a part of the embodiments of the present disclosure but not all of them. Based on the embodiments of the present disclosure, all other embodiments obtained by those of ordinary skill in the art without creative efforts shall fall within the scope of the present disclosure.

The present disclosure is intended to provide an ultrasound catheter for interventional treatment, which integrates the functions of diagnostic intravascular ultrasound (IVUS) imaging and guidewire intervention, and can direct interventional treatment in real time under IVUS guidance and create a channel into the vascular true lumen distal to the occluded section, thereby effectively reducing the operation time and difficulty, and improving the success rate of the operation. The present disclosure is also intended to provide an interventional treatment system comprising the ultrasound catheter for interventional treatment described above.

Now refer to FIGs. 1 to 19. The present application provides an interventional treatment system and an ultrasound catheter thereof for interventional treatment.

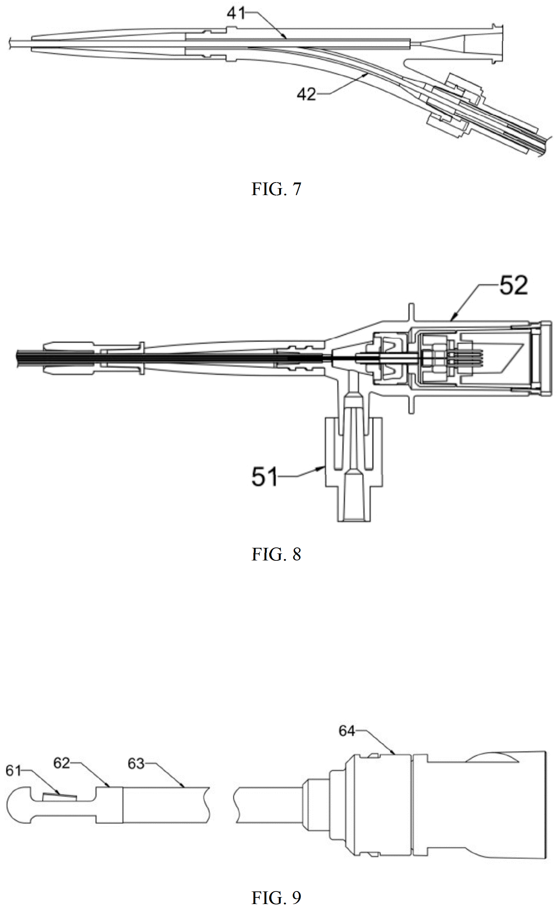

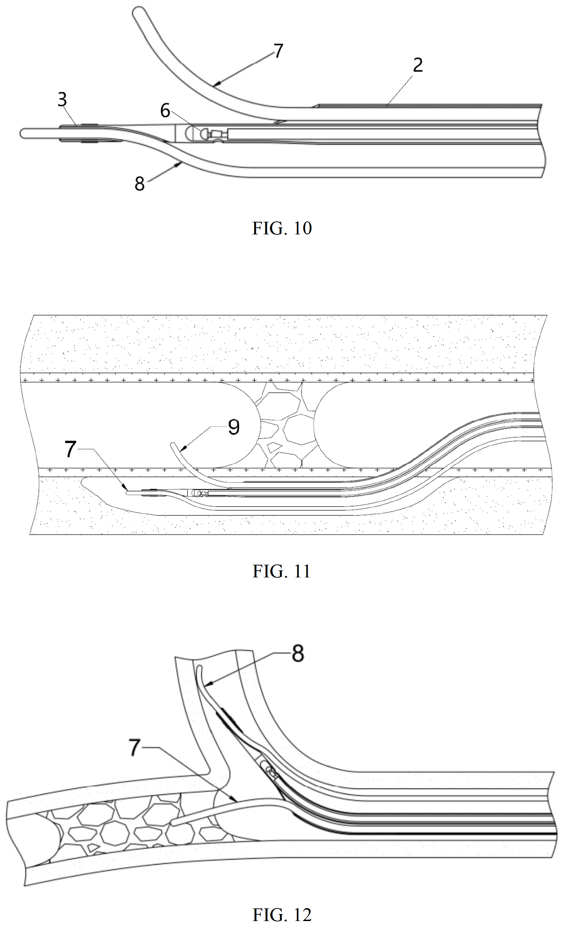

The present application provides an ultrasound catheter for interventional treatment, comprising: a dual-lumen microcatheter 1, comprising a first guide lumen 11 and a first core lumen 12; an acoustically transparent section 2, composed of a material with good acoustically transparent capacity and comprising a second core lumen 21 in communication with the distal end of the first core lumen 12 and a second guide lumen 22 in communication with the distal end of the first guide lumen 11, wherein the second core lumen 21 is configured for accommodating an ultrasound transducer 61 at the distal end of the core 6, the second guide lumen 22 is provided with a guidewire outlet 24, the second guide lumen 22 is a lumen for allowing the exit of an interventional guidewire (such as the second guidewire 7 in FIG. 10) or an interventional treatment ((such as a working guidewire, a balloon, a stent, a rotary milling device, a suction device, etc.)), and the second core lumen 21 and the second guide lumen 22 both extend distally and are arranged in parallel to each other; a rapid exchange section 3, comprising a distal outer tube 31 connected to the distal end of the acoustically transparent section 2, a TIP 32 (i.e., a relatively soft tip at the distal end of the catheter) arranged at the distal end of the distal outer tube 31, and a radiopaque marker ring 33 sheathing the outer peripheral portion of the distal outer tube 31, wherein the rapid exchange section 3 is connected to the distal end of the acoustically transparent section 2, the distal end of the rapid exchange section 3 is configured for allowing the entrance of a directional guidewire (such as the first guidewire 8 in FIG. 10), and the proximal end of the rapid exchange section 3 is configured for allowing the exit of the directional guidewire; and a core 6, provided with an ultrasound transducer 61 arranged at the distal end thereof, wherein the ultrasound transducer 61 has ultrasonographic functionality, the core 6 is arranged within the second core lumen 21 and the first core lumen 12, and the ultrasound transducer 61 is arranged within the second core lumen 21. In some embodiments of the present application, as shown in FIGs. 7 and 8, the ultrasound catheter further comprises: a Y-shaped connection member 4, comprising a third core lumen 42 in communication with the proximal end of the first core lumen 12 and a third guide lumen 41 in communication with the proximal end of the first guide lumen 11; and a connector 5, comprising an irrigation port 51 and a core port 52, both in communication with the third core lumen 42.

The core 6 enters from the core port 52, and reaches the first core lumen 12 through the third core lumen 42 and the second core lumen 21.

The proximal end of the dual-lumen microcatheter 1 is connected to the distal end of the Y-shaped connection member 4, and the third guide lumen 41 and the third core lumen 42 of the Y-shaped connection member 4 are in communication with the first guide lumen 11 and the first core lumen 12 of the dual-lumen microcatheter 1, respectively. The Y-shaped connection member 4 is connected to the connector 5 through the third core lumen 42 and is in communication with the irrigation port 51 and the core port 52. In the practical application process, the shape, structure, size, material, position, and the like of the dual-lumen microcatheter 1, the acoustically transparent section 2, the rapid exchange section 3, the Y-shaped connection member 4, the connector 5, the core 6, the second guidewire 7, and the first guidewire 8 can be determined according to actual conditions and actual requirements.

In addition to the ultrasound catheter for interventional treatment described above, the present application further provides an interventional treatment system comprising the ultrasound catheter for interventional treatment described above. The interventional treatment system comprises a number of guidewires and the ultrasound catheter for interventional treatment described above. The guidewires include an interventional guidewire extending out through the guidewire outlet 24 and a directional guidewire passing through the rapid exchange section 3. The directional guidewire is configured for directing the ultrasound catheter to a desired position, and the interventional guidewire is configured for delivering to a target position under the imaging guidance of the ultrasound catheter. The guidewire can be switched between a status of extending out through the guidewire outlet 24 and a status of passing through the rapid exchange section 3, such that the guidewire can be switched between the directional guidewire and the interventional guidewire.

In contrast, in the prior art, conventional catheters with ultrasound capabilities are of single-lumen structure designed exclusively for ultrasound transducer imaging. They possess limited functionality and cannot directly achieve therapeutic effects. The present application provides an ultrasound catheter with dual lumens, and by utilizing the characteristic that the dual-lumen catheter can be equipped with functions of ultrasonography and interventional treatment, various treatment purposes can be met by taking ultrasonographic direction as a platform.

Compared with existing devices integrating interventional treatment with ultrasonographic functionality, the dual-lumen structure has a smaller radial dimension and a broader application range, and can be conveniently and flexibly integrated into multiple types of blood vessels for interventional treatment. Since interventional treatment devices do not need to be connected in series on the same first guidewire to reach the target position, the synchronous operation of ultrasonographic and the interventional treatment devices can be conveniently achieved. Particularly, if the interventional treatment device enters another branch, the ultrasound images cannot continue to follow for real-time ultrasonographic direction. Especially for operations with complex procedures, multiple devices connected in series on one first guidewire require a strict design sequence; otherwise, the unnecessary retraction of devices from the body and the rearrangement of the devices must be performed multiple times, making the operation complicated, significantly limiting the application scenes, and also making it difficult to ensure the continuity of ultrasonographic direction. The dual-lumen structure can conveniently maintain the synchronization between the core 6 and the interventional treatment devices in another lumen without requiring a serial connection design on the directional guidewire, thereby greatly simplifying the procedures, reducing the surgical difficulty, and helping improve the success rate of the operation.

In the following embodiments, an exemplary interventional treatment device with ultrasonographic functionality for occlusion interventional treatment is used for illustration. It will be appreciated that the scope of the present application includes but is not limited to this embodiment.

In the treatment of occlusive lesions, for example, chronic total occlusion (CTO) lesions, effectively recanalizing the occluded section or establishing a path through the occluded section is a key to surgical success. In clinical practice, due to hard fiber caps at the occlusion site, the guidewire often cannot recognize the occlusion entrance suitable for intervention, or the direction of the guidewire cannot be controlled and the guidewire may mistakenly enter the interlayer between the intima and media to form a false lumen. The prior art lacks the function of real-time imaging directions, making it difficult to distinguish the direction of the intima and the occluded section and to effectively manipulate a guidewire to cross the intima and re-enter the true lumen or find the entrance of vascular occlusion. Due to the possibility of "blind puncturing", multi-posture alignment imaging may be required, which will result in prolonged operation time and decreased surgical success rate; moreover, the expensive surgical devices impose an economic burden on patients.

It should be noted that according to the present application, a certain guidewire is not merely limited to an interventional guidewire or a directional guidewire, but may serve as an interventional guidewire or a directional guidewire in a certain implementation stage and serve as the other one in the next stage by adjusting the connection relationship between the guidewire and the ultrasound catheter. Therefore, the guidewire can be switched between the interventional guidewire and the directional guidewire. For example, in the treatment of an occlusive lesion, after the ultrasound catheter reaches the true lumen of the intima proximal to the occlusive section along the first guidewire 8, the second guidewire 7 penetrates into the subintimal cavity under the direction of the ultrasound catheter. Then the ultrasound catheter and the first guidewire 8 are retracted. Under the direction of the second guidewire 7, the second guidewire 7 enters the rapid exchange section 3 of the ultrasound catheter, the ultrasound catheter is pushed to the subintimal cavity along the second guidewire 7, and the third guidewire 9 is delivered by means of the first guide lumen 11 and the second guide lumen 22. The third guidewire 9 penetrates through the intima, returns to the true lumen of the intima under the imaging direction of the ultrasound catheter, and reaches the vascular true lumen distal to the occluded section.

It can be seen from the above that the directional guidewire may include the first guidewire 8 and the second guidewire 7, and the interventional guidewire may include the second guidewire 7 and the third guidewire 9. It can be seen that the second guidewire 7 may be either a directional guidewire or an interventional guidewire depending on the implementation stage. When it exerts a directional function in the current implementation stage, it is considered a directional guidewire for that stage; when it exerts an interventional (e.g., puncturing, inspecting) function in the current implementation stage, it is considered an interventional guidewire. The function performed by the guidewire in the current stage has no necessary connection with its function in the next stage, and can instead be flexibly adjusted according to the implementation objective. The aforementioned description is merely an example from the perspective of ease of understanding, and there may be various specific embodiments in practical implementation according to actual needs. For example, the same stiff guidewire is used for each puncture through the vascular intima, then retracted after completing the puncture; then a softer guidewire is introduced through the puncture tract into the false lumen and back to the true lumen. All possible variations are not exhaustively listed here.

In addition, the target position described in the present application is not a constant position, but may be a preferred intervention position selected for the ultrasound catheter according to the ultrasonography at different stages. In a preferred condition, the operator can introduce the first guidewire 8 into the patient's body. The first guidewire 8 passes through the occluded section and reaches the true lumen at the distal end of the occluded section. The first guidewire 8 provides a push path for an interventional treatment device (e.g., a working guidewire, a balloon, a stent, a rotary milling device, a suction device, etc.), enabling the interventional treatment device to operate near the occluded section and thus establishing a passage for the interventional treatment device to clear the occluded section. For instance, the obstructive material within the occluded section may be made looser via balloon dilation or may be milled using the rotary milling device along the first guidewire 8, among other manners, to establish the passage. Additional interventional treatment devices can be adaptively employed according to the requirements, and will not be elaborated herein.

In general, it is difficult for an operator to directly find the occlusion entry and puncture the entry with the guidewire by blind puncturing. In some embodiments, as shown in FIG. 12, when the first guidewire 8 enters a branch vessel of the vascular occluded section, the ultrasound catheter is delivered along this first guidewire 8. Then, the second guidewire 7 is delivered by means of the third guide lumen 41 and the first guide lumen 11. The second guidewire 7 exits through the guidewire outlet 24 of the second guide lumen 22, and under the real-time IVUS direction, finds the occlusion entrance and reaches the distal end of the occluded section, such that the ultrasonographic function of the core 6 can be started. The relative position of the second guidewire 7 and the occlusion entrance is observed in real time. When the artifact of the second guidewire 7 is determined to be located on the same side as the occlusion entrance, the second guidewire 7 is operated to enter the occlusion section and allow the distal end of the second guidewire 7 to reach the vascular true lumen distal to the occluded section through continuous delivery.

It can be seen from the description above that, in the present application, the IVUS function of the ultrasound catheter saves the operation time for finding the occlusion inlet, and the operator can adjust the first guidewire 8 with a more appropriate hardness according to the image requirements of the occluded section visualized by the ultrasound catheter. The ultrasound catheter of the present application can effectively reduce the operation time and difficulty and improve the success rate of the operation.

The occluded vascular section is generally a calcification fiber cap. When the occluded vascular section is relatively denser and has no occlusion inlet, or the occluded vascular section has an occlusion inlet but a guidewire cannot be delivered to the vascular true lumen distal to the occluded section, the occluded vascular section needs to be bypassed to reach the vascular true lumen distal to the occluded section. In this case, how to penetrate from the true lumen at the proximal end of the vascular occlusion into the subintimal false lumen and how to re-enter the vascular true lumen at the distal end of the vascular occlusion after entering the subintimal false lumen are difficulties that need to be addressed and overcome.

In some embodiments, the ultrasound catheter of the present application may reach the proximal end of the occluded vascular section via the first guidewire 8, and the ultrasonographic function of the ultrasound catheter can direct the second guidewire 7 to penetrate into the false lumen beneath the intima from the vascular true lumen. Since the shape of the occluded vascular section is generally irregular, real-time imaging direction can help identify a puncture site that is more suitable for penetrating into the subintimal cavity. By targeting this puncture site, the distal end of the second guidewire 7 can be directed to enter beneath the intima.

Then, the ultrasound catheter and the first guidewire 8 are retracted. Under the direction of the second guidewire 7, the second guidewire 7 enters the rapid exchange section 3 of the ultrasound catheter, the ultrasound catheter is pushed along the second guidewire 7 to the subintimal cavity, and the third guidewire 9 is delivered through the first guide lumen 11 and the second guide lumen 22. As shown in FIG. 11, under the real-time direction of the IVUS function, the puncture site that is easier for puncturing and returning to the true lumen can be identified through the real-time imaging direction. The guidewire outlet 24 of the second guide lumen 22 allows the exit of the third guidewire 9, and the third guidewire 9 travels through the intima and returns to the vascular true lumen. The distal end of the third guidewire 9 can penetrate back to the target position at the distal end of the occluded section, such that the third guidewire 9 creates a path crossing the occluded section from the true lumen at the proximal end of the occluded section to the true lumen at the distal end of the occluded section.

In addition, in the case that the distal end of the first guidewire 8 mistakenly enters the subintimal false lumen (not shown), the ultrasound catheter may be further pushed into the false lumen along the first guidewire 8 and penetrate back to the distal end of the occluded section according to the aforementioned embodiment, which will not be recited herein. The third guidewire 9 is an interventional treatment guidewire, i.e., a guidewire that ultimately establishes an occlusive lesion treatment channel across the stenosis.

In the present application, the ultrasonographic function of the core 6 is started, and the relative position of the second guidewire 7 and the vascular true lumen is observed in real time. When it is determined that the artifact of the second guidewire 7 and the vascular true lumen are located on the same side, it indicates that the puncture direction is correct, and the second guidewire 7 is operated to penetrate through the intima and enter the vascular true lumen distal to the occlusion section; if the artifact of the second guidewire 7 and the vascular true lumen are not on the same side, the ultrasound catheter needs to be rotated to rotate the position of the guidewire outlet 24 of the second guide lumen 22 to a position on the same side as the vascular true lumen, and then the pushing and puncture of the second guidewire 7 are performed. Thereafter, the first guidewire 8 and the ultrasound catheter are retracted, leaving only the second guidewire 7 in the patient's body to provide a push path for the interventional treatment devices.

The implementation principle that the ultrasound catheter can direct the second guidewire 7 to directionally penetrate into the subintimal false lumen by means of the ultrasonographic function is the same as the aforementioned implementation principle that the second guidewire 7 can be directed to directionally penetrate back into the vascular true lumen by means of the ultrasonographic function, and the embodiments are similar. Those skilled in the art can understand the implementation according to FIG. 11, which will not be further elaborated.

As can be seen from the above, the present application selects proper puncture sites for entering the false lumen and returning to the true lumen through the IVUS function of the ultrasound catheter, thereby effectively creating a path bypassing the occluded vascular section. It can also remedy the situation where the first guidewire accidentally enters the vascular false lumen, where the first guidewire 8 that has mistakenly entered the false lumen is substituted with the second guidewire 7 that enters the true lumen. The ultrasound catheter of the present application simplifies the ADR (antegrade dissection and re-entry) technique and improves the success rate of the operation.

It should be noted that the second guidewire 7 and the third guidewire 9 described above may be substituted with more flexible guidewires, and the forms described above for establishing a channel by direct puncture are only illustrative. For example, to avoid puncturing the vascular membrane or causing hematoma after the second guidewire 7 with higher rigidity enters the subintimal cavity, the second guidewire 7 may exit after forming a puncture tract on the vascular membrane, and be replaced with a more flexible working guidewire (i.e., the third guidewire 9) that enters the subintimal cavity or penetrates out of the subintimal cavity through the puncture tract.

Alternatively, the above-mentioned second guidewire 7 may also be directly substituted with an interventional treatment device. For example, a balloon is delivered through the first guide lumen 11 and the second guide lumen 22 and enters the target position under the direction of the ultrasound transducer 61 or dilates at the target position. This is not limited in the present application, and an option of embodiments or any combination of multiple embodiments made by operators or those skilled in the art according to the actual needs shall fall within the scope of the present application.

In addition to establishing a path from the true lumen at the proximal end of the occluded section to the true lumen at the distal end of the occluded section in the embodiments described above, the ultrasound catheter of the present application can also be used to create a vascular pathway alongside the occluded vascular section. The interlayer between the intima and media of the vascular section where the occlusion occurs is utilized as a new blood flow path, connecting the proximal end to the distal end of the occluded vascular section to form a bypass blood flow channel around the occluded vascular section.

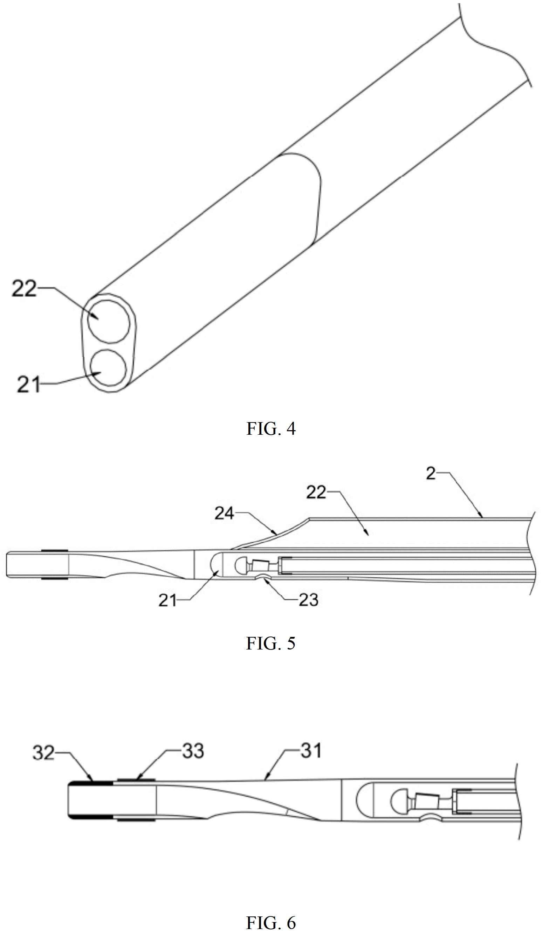

In order to improve the single-lumen ultrasound catheter to the dual-lumen ultrasound catheter according to the present application, the second guide lumen 22 is arranged alongside the second core lumen 21 in a substantially parallel manner, and the outer profile of the acoustically transparent section 2 is elliptical or circular, or the outer profile at the distal end of the acoustically transparent section 2 is elliptical and the outer profile at the proximal end is circular, and the outer profile of the middle section of the acoustically transparent section 2 transitions from the circular shape to the elliptical shape. The aforementioned elliptical shape may be a regular elliptical shape, or may be, for example, a cobblestone-like irregular elliptical shape. The profile of the elliptical shape being a regular elliptical shape or an irregular elliptical shape mainly depends on the ratio of the radial dimensions of the second guide lumen 22 to the second core lumen 21, as shown in FIG. 4.

When the second guidewire 7 penetrates into the intima or back into the true lumen as directed by the ultrasonography, the second guide lumen 22 is located between the second core lumen 21 and the target position (a proper puncture position for performing puncture to enter the subintimal cavity or a proper puncture position for performing puncture back to the true lumen). Therefore, the ultrasound transducer 61 in the second core lumen 21 needs to acquire the dynamics of the relative positions between the second guidewire 7 extended out through the guidewire outlet 24 of the second guide lumen 22 and the target position in real time.

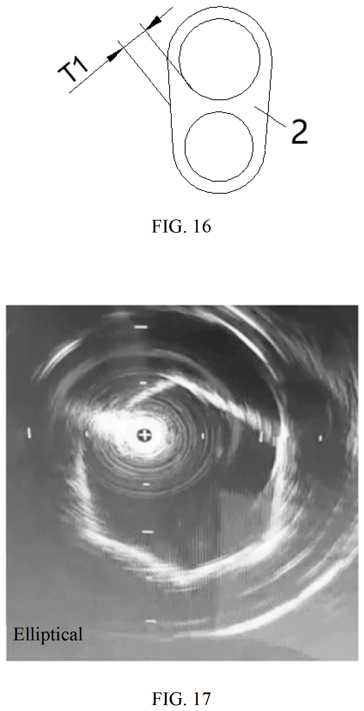

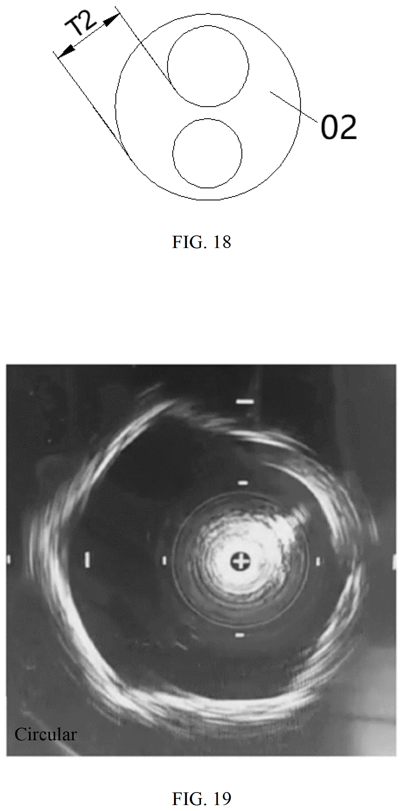

In some embodiments, in order to avoid affecting the ultrasonographic function, the acoustically transparent section 2 is preferably provided with a regular or irregular elliptical profile, such that the thickness of the acoustically transparent material around the ultrasound transducer 61 can be significantly reduced, the ratio of the minor axis to the major axis of the elliptical profile is 1:1.7 to 1:2.3, and the ratio of the minimum wall thickness to the maximum wall thickness of the elliptical profile is 1:4 to 1:6. The maximum wall thickness T1 of the elliptical profile is about 1/4 to 1/2 of the maximum wall thickness T2 of circular profile, as shown in FIGs. 16 and 17. The ratio of the minimum wall thickness to the maximum wall thickness of the circular outer profile is 1:8 to 1:24, as shown in FIGs. 18 and 19.

Compared with an acoustically transparent section 2 with a circular profile 02, an acoustically transparent section 2 with an elliptical profile is adopted in the present application. The reduction of the wall thickness of the acoustically transparent section 2 improves the ultrasonic transmissivity, thereby achieving better definition and detection depth and a wider field of view. The imaging in the hexagonal phantom can obtain a complete hexagonal image, as shown in FIG. 17. In contrast, the circular acoustically transparent section displays an image with lines that are not straight and clear enough, as shown in FIG. 19.

In some embodiments, the first core lumen 12 and the second core lumen 21 are both cylindrical cavities, and the core 6 can move forwards or backwards relative to the first core lumen 12 and the second core lumen 21; and the ultrasound transducer 61 has an initial position substantially flush with the guidewire outlet 24, and the ultrasound transducer 61 has a detection position when moving relative to the guidewire outlet 24.

In some embodiments, the core 6 is arranged in the first core lumen 12 and the second core lumen 21, where both the first core lumen 12 and the second core lumen 21 are cylindrical cavities, allowing the core 6 to rotate forward or backward. Since both the first core lumen 12 and the second core lumen 21 of the apparatus are cylindrical cavities, when the core 6 rotates forward or backward, the first core lumen 12 and the second core lumen 21 do not impede the movement of the core 6, facilitating smooth forward or backward movement of the core 6 and improving the alignment and direction of the ultrasound transducer 61 and the guidewire outlet 24. Moreover, the detection position here may be any position other than the initial position during the retraction process.

In some embodiments, specifically, the core 6 has a retraction function. During the operation, the IVUS function is activated to observe the structural condition of the diseased blood vessel. The distal end of the core in the initial state (the position of the ultrasound transducer 61) is substantially flush with the exit position of the second guidewire 7. Then, the core 6 is retracted, and the blood vessel section corresponding to the retraction distance shall include both an un-occluded section and an occluded section as much as possible, thereby ensuring that the initial position of the distal end of the core 7 is in the un-occluded section and at a relatively suitable position from the occluded section, and a more proper puncture point can be selected for the second guidewire 7. Compared with other types of transducers such as a multi-array element or a ring array, the ultrasound transducer 61 capable of rotating forward or backward can achieve the retraction function and reduce the dimension, thus making the diameter of the ultrasound catheter smaller.

In order to reduce the extent of tear under the intima and to reduce hematomas, excessive undesirable separation of the intima from the media in the blood vessel in regions outside the field of view of the ultrasound transducer 61 shall be avoided as the rapid exchange section 3 is arranged at the distal end of the acoustically transparent section 2.

In some embodiments, the length L1 of the rapid exchange section 3 is 1/5 to 1/7 of the distance (L1 + Lt) from the distal end of the entire ultrasound catheter to the proximal end of the acoustically transparent section, and the rapid exchange length L1 of the rapid exchange section 3 is 1/4 to 1/2 of the length L2 of the RX rapid exchange section 03 in commercially available IVUS products. That is, by shortening the length of the rapid exchange section 3, the blind area in the visual field becomes smaller, resulting in reduced vascular interlayers. When the rapid exchange section 3 is located beneath the vascular intima, the caused vascular interlayer is also minimized, which helps alleviate hematomas and increase the operator's puncture success rate. In addition to the effects noted above, minimizing the dimensions of the rapid exchange section 3 is also beneficial to transmitting torsional force, thereby preventing kinking of the ultrasound catheter.

As shown above, the ultrasound catheter has a state of penetrating beneath the intima and penetrating back to the true lumen, that is, the ultrasound catheter as a whole will have a state crossing the intima, where a part of the ultrasound catheter is in the false lumen beneath the intima, and another part is in the vascular true lumen. As described above, if the guidewire outlet 24 is not on the same side as the target position, the ultrasound catheter needs to be rotated to position the two on the same side. Minimizing the dimensions of the rapid exchange section 3 will improve the torsional force and the compliance and torque transmission capacity from the proximal end of the ultrasound catheter to the rapid exchange section 3, and prevent bending.

In some embodiments, the acoustically transparent section 2 is provided with an arc-shaped profile at the guidewire outlet 24, such that the radial dimension of the acoustically transparent section 2 gradually decreases as it extends distally, thereby avoiding a sudden change in the radial dimension of the acoustically transparent section 2, and enabling better transmission of a torsional force and better response to the torsional force of the ultrasound catheter without kinking. Also, the guidewire outlet 24 is provided with an arc-shaped profile, which can allow the second guidewire 7 or the interventional medical device extending out through the guidewire outlet 24 to have a greater bending angle.

In some embodiments, an arc-shaped hole may be provided in the distal outer tube 31 of the rapid exchange section 3. When it is necessary to push an ultrasound catheter along the first guidewire 8, the first guidewire 8 enters through the distal end of the TIP 32, moves along the arc-shaped hole of the distal outer tube 31, and finally exits at the proximal end of the distal outer tube 31. The arc-shaped hole can ensure a greater contact area between the first guidewire 8 and the rapid exchange section 3, making the pushing operation smoother.

In summary, the ultrasound catheter for an interventional treatment device or a guidewire provided herein, which integrates the functions of diagnostic intravascular ultrasound (IVUS) imaging and guidewire treatment, can direct the intervention in real time under the direction of IVUS, improve the success rate of intervention at the target position, effectively reduce the operation time and difficulty, and improve the success rate of the operation. In some embodiments, the acoustically transparent section 2 comprises a liquid outlet 23 and a guidewire outlet 24. The liquid outlet 23 is located on a reverse side of the second guide lumen 22, and the guidewire outlet 24 is located at the distal end of the second guide lumen 22.

It should be noted that the second core lumen 21 and the second guide lumen 22 of the acoustically transparent section 2 may contain a liquid to improve the imaging performance of the apparatus and reduce the influence on the ultrasonography. The arrangement of the liquid outlet 23 on the reverse side (opposite side) of the second guide lumen 22 is beneficial to discharging the liquid.

The guidewire outlet 24 is provided at the distal end of the second guide lumen 22. In some embodiments, the distal end of the second guide lumen 22 is parallel to the second core lumen, and the curvature of the second guidewire 7 itself can be utilized, such that the second guidewire 7 has a certain curvature after penetrating out. In addition, the distal end of the second guide lumen 22 may also be provided with an elbow structure, such that the second guidewire 7 has a certain curvature after penetrating out of the distal end of the second guide lumen 22.

In some embodiments, as shown in FIG. 9, the core 6 comprises a transducer base 62, an ultrasound transducer 61 provided on the transducer base 62, a torque spring 63, and a core connector 64. The transducer base 62 is connected to one end of the torque spring 63, and the other end of the torque spring 63 is connected to the core connector 64. The core 6 enters through the core port 52 of the connector 5 and enters the second core lumen 21 of the acoustically transparent section 2 through the third core lumen 42 of the Y-shaped connection member 4. Moreover, the ultrasound transducer 61 can reach the distal end of the second core lumen 21 of the acoustically transparent section 2; that is, the position of the ultrasound transducer 61 aligns with the position of the guidewire outlet 24 of the acoustically transparent section 2.

In some embodiments, the distal end of the dual-lumen microcatheter 1 is connected to the proximal end of the acoustically transparent section 2 by welding; that is, the acoustically transparent section 2 and the dual-lumen microcatheter 1 are integrally welded, wherein the second core lumen 21 and the second guide lumen 22 of the acoustically transparent section 2 are in communication with the first core lumen 12 and the first guide lumen 11 of the dual-lumen microcatheter 1, respectively. The distal end of the acoustically transparent section 2 is connected to the proximal end of the rapid exchange section 3 by welding. That is, the acoustically transparent section 2 and the rapid exchange section 3 are integrally connected by thermal welding.

In some embodiments, the outer layer of the dual-lumen microcatheter 1 is a polymer material member that gradually decreases in hardness from the proximal end to the distal end, and the polymer material is one or more of PEBAX, TPU, and PE. The middle layer of the dual-lumen microcatheter 1 is a metal braid and/or coil made of one or more of stainless steel, nickel-titanium alloy, platinum-iridium alloy, a liquid crystal polymer, and a polymer material.

It should be noted that when the dual-lumen microcatheter 1 is formed by combining multiple layers of Reflow, the material of the inner dual-lumen tube of the dual-lumen microcatheter 1 is selected from one or more of PEBAX, PTFE, HDPE, and TPU. The middle layer structure of the dual-lumen microcatheter 1 is a braid or/and a coil, and the material is selected from one or more of metals or polymers such as stainless steel, nickel-titanium alloy, platinum-iridium alloy, LCP, and the like. The material of the outer layer of the dual-lumen microcatheter 1 is selected from one or more of PEBAX, TPU, PE, and the like.

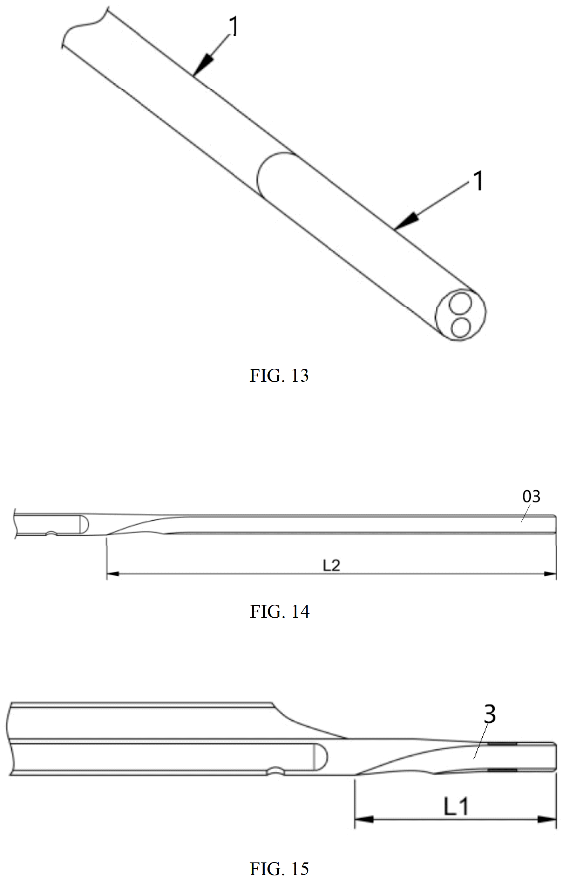

One processing technique is as follows: two mandrels are each sheathed with an ultra-thin PTFE tube, and together pass through the metal-braided mesh tube. Subsequently, the outer layer of the metal-braided mesh tube is sheathed with two PEBAX single-lumen tubes, with the hardness of the single-lumen tubes gradually decreasing from proximal to distal, which is from 72D to 55D. Finally, the outermost layer is covered with a FEP heat shrink tube, and hence the assembly is welded in segments to form an integral structure. After the process is completed, the FEP heat shrink tube is torn off, and the two mandrels are drawn out to obtain the product. That is, the dual-lumen microcatheter 1 can be formed by integral coating and extrusion or by thermally combining an inner polymer dual-lumen tube, a middle spring/braid layer, and an outer resin layer (Reflow), where the outer resin layer is welded in segments and gradually decreases in hardness from proximal to distal. The structure is shown in FIG. 13, and the two segments of the dual-lumen microcatheter 1 differ in hardness.

In some embodiments, the inner layers of the first guide lumen 11 and the first core lumen 12 are both made of one or more of PEBAX, PTFE, HDPE, and TPU, that is, the first guide lumen 11 and the first core lumen 12 are polymer materials with a relatively low coefficient of friction. The inner walls of the first guide lumen 11 and the first core lumen 12 are both provided with a PTFE ultralow-friction layer. In addition, the inner walls of the first core lumen 12 and the second guide lumen 22 of the acoustically transparent section 2 are not provided with the PTFE ultralow-friction layer. In some embodiments, the acoustically transparent section 2 is made of PE or Pebax, so as to ensure the good sound transmission effect of the acoustically transparent section 2.

In some embodiments, the distal outer tube 31 is made of Pebax or PA; the TIP 32 is made of Pebax or PU; the radiopaque marker ring 33 is made of platinum-iridium alloy, tantalum, or gold. Moreover, the proximal end of the distal outer tube 31 may be welded integrally with the distal end of the acoustically transparent section 2, and the distal outer tube 31 is located at the reverse side of the second guide lumen 22.

In some embodiments, the interventional treatment system further comprises an interventional medical device, and the interventional medical device includes at least one of a working guidewire, a balloon, a stent, a rotary milling device, and a suction device;

The interventional medical device is configured for delivering along the second guidewire 7 to a target position, or alternatively, the interventional medical device is configured for extending out through the guidewire outlet 24 and delivering to the target position directly.

In some embodiments, the interventional treatment system is configured for interventional occlusion treatment; the guidewires further include a third guidewire 9 for interventional treatment; the distal end of the third guidewire 9 is arranged at the distal end of an occluded vascular section, the proximal end of the third guidewire 9 is arranged at the proximal end of the vascular occlusion section, and a middle section of the third guidewire 9 bypasses the occluded vascular section under the imaging guidance of the ultrasound catheter through an interlayer between the intima and media of the occluded vascular section. The third guidewire 9 is an interventional treatment guidewire, i.e., a guidewire that ultimately establishes an occlusive lesion treatment channel across the stenosis.

It should be noted that the first guide lumen 11, the second guide lumen 22, and the guidewire outlet 24 of the ultrasound catheter allow a guidewire to sequentially pass through, and the rapid exchange section 3 of the ultrasound catheter allows a guidewire to pass through. The guidewire is the second guidewire 7, the first guidewire 8, or the third guidewire 9 as described in the foregoing embodiments. That is, the first guidewire 8, the second guidewire 7, and the third guidewire 9 may have the specific features of any embodiments above. The first guide lumen 11, the second guide lumen 22, and the third guide lumen 41 are configured for allowing the second guidewire 7 or the third guidewire 9 to pass through, and the rapid exchange section 3 may allow the first guidewire 8 or the second guidewire 7 to pass through. In some embodiments, the second guidewire 7 or the third guidewire 9 may sequentially pass through the third guide lumen 41 of the Y-shaped connection member 4, the first guide lumen 11 of the dual-lumen microcatheter 1, and the second guide lumen 22 of the acoustically transparent section 2, and finally exit through the guidewire outlet 24 of the acoustically transparent section 2.

In some embodiments, the first guidewire 8 or the second guidewire 7 enters through the distal end of the TIP 32 and exits through the proximal end of the distal outer tube 31. The first guidewire 8 and the second guidewire 7 can create a push path for the ultrasound catheter and allow the ultrasound catheter to be pushed along the first guidewire 8 and the second guidewire 7 to the target position.

It should be noted that in all the aforementioned specific embodiments, the end proximal to the operator (e.g., a healthcare provider) is defined as the proximal end, and the end for extending into the patient is defined as the distal end. The end of a single member proximal to the operator is the proximal end and the end distal to the operator is the distal end.

For example, in practical use, although the distal outer tube is entirely disposed within the patient, the proximal end of the distal outer tube refers to the end or side proximal to the operator along the path of catheter pushing intervention. In the embodiment as shown in FIG. 1 or FIG. 6, the direction of catheter pushing intervention is from the right side of the figure to the left side of the figure, and the proximal end of the distal outer tube is near the joint of the distal outer tube and the acoustically transparent section 2; that is, the proximal end of the distal outer tube is on the right side of the distal outer tube in the figure. The distinction between the distal end and the proximal end of the other components is similar to that in the aforementioned description unless otherwise explicitly excluded.

In addition, for the first guide lumen 11, the second guide lumen 22, the third guide lumen 41, the first core lumen 12, the second core lumen 21, the third core lumen 42, the first guidewire 8, the second guidewire 7, and the third guidewire 9 described herein, the "first", "second", and "third" are merely used for distinguishing positional differences and do not imply the order or sequence.

In addition, it should also be noted that directions or positional relationships indicated by "proximal/distal" or the like in the present disclosure are based on the directions or positional relationships shown in the drawings, and are merely intended to simplify description and facilitate understanding rather than to indicate or imply that the indicated apparatus or element must have a specific direction and be configured and operated according to the specific direction. Therefore, such directions or positional relationships should not be construed as limiting the present disclosure. For convenience of description, only parts associated with the related invention are shown in the accompanying drawings.

The embodiments and features of the embodiments described below can be combined in the case of no conflicts. As used in the present disclosure, the terms "mount", "link", "connect", and one element being "arranged" relative to another element, should be interpreted in their broad senses and generally, only indicate that there is a connection, coupling, fitting, or transmission relationship between the two elements, and the two elements may be in connection, coupling, fitting, or transmission directly or indirectly via an intermediate element, or the two elements may be connected by means of energy or signal response, and should not be construed as indicating or implying a spatial position relationship or a direct contact relationship between the two elements; that is, one element may be optionally located inside, outside, above, below, or to one side of the other element, unless otherwise explicitly indicated in the content. For those of ordinary skill in the art, the specific meanings of the aforementioned terms in the present disclosure can be interpreted according to specific conditions.

In the present specification, the embodiments are described in a progressive manner. Each example focuses on differences from other embodiments, and the same and similar parts among the embodiments can refer to each other. Any combination of the embodiments provided herein shall fall within the scope of the present disclosure, which will not be further recited herein.

The interventional treatment system and the ultrasound catheter for interventional treatment provided herein are described in detail above. The principle and implementations of the present disclosure are described with specific embodiments herein. The foregoing embodiments are merely intended to help understand the method and core idea of the present disclosure. It should be noted that for those of ordinary skill in the art, without departing from the principle of the present disclosure, various improvements and modifications can also be made to the present disclosure, and such improvements and modifications shall also fall within the scope of the claims of the present disclosure.

## Claims

1. An ultrasound catheter for interventional treatment, comprising:
a dual-lumen microcatheter, comprising a first guide lumen and a first core lumen;
an acoustically transparent section, comprising a second core lumen in communication with the distal end of the first core lumen and a second guide lumen in communication with the distal end of the first guide lumen, the second guide lumen being provided with a guidewire outlet, and the second core lumen and the second guide lumen both extending distally and being arranged in parallel to each other;
a core, provided with an ultrasound transducer arranged at the distal end thereof, the core being arranged within the second core lumen and the first core lumen, and the ultrasound transducer being arranged within the second core lumen; and
a rapid exchange section, connected to the distal end of the acoustically transparent section, the distal end of the rapid exchange section being configured for allowing the entrance of a directional guidewire, and the proximal end of the rapid exchange section being configured for allowing the exit of the directional guidewire,
wherein, the ultrasound catheter for interventional treatment further comprises a Y-shaped connection member and a connector;
the Y-shaped connection member comprises a third core lumen in communication with the proximal end of the first core lumen and a third guide lumen in communication with the proximal end of the first guide lumen;
the connector comprises an irrigation port and a core port, both in communication with the third core lumen.

2. The ultrasound catheter for interventional treatment according to claim 1, wherein the ultrasound transducer is configured for selecting a target position, the ultrasound transducer is configured for acquiring an image in a radial direction of the second core lumen, and the ultrasound catheter can be rotated such that the guidewire outlet and the target position are arranged on the same side of the ultrasound transducer.

3. The ultrasound catheter for interventional treatment according to claim 1, wherein the acoustically transparent section is made of a material that is readily transparent to sound;
the cross-sectional profile of the acoustically transparent section is circular, or the cross-sectional profile of the acoustically transparent section transitions from circular to elliptical as the acoustically transparent section extends distally.

4. The ultrasound catheter for interventional treatment according to claim 1, wherein the cross-sectional profile of the acoustically transparent section is elliptical; and
the ratio of a minor axis to a major axis of the elliptical acoustically transparent section is 1:1.7 to 1:2.3, and/or the ratio of a minimum wall thickness to a maximum wall thickness of the elliptical acoustically transparent section is 1:4 to 1:6.

5. The ultrasound catheter for interventional treatment according to any one of claims 1 to 4, wherein the first core lumen and the second core lumen are both cylindrical cavities, and the core can move forwards or backwards relative to the first core lumen and the second core lumen; and
the ultrasound transducer has an initial position substantially flush with the guidewire outlet, and the ultrasound transducer has a detection position when moving relative to the guidewire outlet.

6. The ultrasound catheter for interventional treatment according to any one of claims 1 to 4, wherein the rapid exchange section has a length L1, the distance from the distal end of the ultrasound catheter to the proximal end of the acoustically transparent section is (L1 + Lt), and the ratio of L1 to (L1 + Lt) is 1/5 to 1/7.

7. The ultrasound catheter for interventional treatment according to any one of claims 1 to 4, wherein the acoustically transparent section is provided with an arc-shaped profile at the guidewire outlet, and the radial dimension of the acoustically transparent section gradually tapers as the acoustically transparent section extends distally; and/or
the rapid exchange section is provided with an arc-shaped hole for allowing the directional guidewire to pass through in a bent state.

8. The ultrasound catheter for interventional treatment according to any one of claims 1 to 4, wherein the dual-lumen microcatheter comprises an inner polymer layer, an intermediate reinforcement layer, and an outer resin layer in sequence from inside to outside;
the inner polymer layer is made of a polymer material with a low coefficient of friction, and the inner polymer layer forms the first guide lumen and the first core lumen;
the intermediate reinforcement layer is formed by braiding or winding around or sheathing the inner polymer layer;
the outer resin layer is made of at least two materials with different hardnesses, and the hardness of the outer resin layer gradually decreases from the proximal end to the distal end;
the inner polymer layer, the intermediate reinforcement layer, and the outer resin layer are formed into the ultrasound catheter by the co-extrusion technology or heat shrink tubing technology.

9. An ultrasound catheter for interventional treatment, comprising:
a dual-lumen microcatheter, comprising a first guide lumen and a first core lumen;
an acoustically transparent section, comprising a second core lumen in communication with the distal end of the first core lumen and a second guide lumen in communication with the distal end of the first guide lumen, the second guide lumen being provided with a guidewire outlet, and the second core lumen and the second guide lumen both extending distally and being arranged in parallel to each other;
a core, provided with an ultrasound transducer arranged at the distal end thereof, the core being arranged within the second core lumen and the first core lumen, and the ultrasound transducer being arranged within the second core lumen; and
a rapid exchange section, connected to the distal end of the acoustically transparent section, the distal end of the rapid exchange section being configured for allowing the entrance of a directional guidewire, and the proximal end of the rapid exchange section being configured for allowing the exit of the directional guidewire.

10. The ultrasound catheter for interventional treatment according to claim 9, wherein the proximal end of the ultrasound catheter optionally allows the entrance of interventional guidewires with different hardnesses, and the interventional guidewire sequentially passes through the first guide lumen of the dual-lumen microcatheter and the second guide lumen of the acoustically transparent section, such that the interventional guidewires with different hardnesses extend out through the guidewire outlet;
the ultrasound catheter can exit a true lumen relative to a guidewire and enter a subintimal cavity under the direction of the guidewire, so as to switch the guidewire from the interventional guidewire to the directional guidewire.

11. The ultrasound catheter for interventional treatment according to claim 9, wherein the ultrasound transducer is configured for selecting a target position, the ultrasound transducer is configured for acquiring an image in a radial direction of the second core lumen, and the ultrasound catheter can be rotated such that the guidewire outlet and the target position are arranged on the same side of the ultrasound transducer.

12. The ultrasound catheter for interventional treatment according to claim 9, wherein the acoustically transparent section is made of a material that is readily transparent to sound;
the cross-sectional profile of the acoustically transparent section is circular, or the cross-sectional profile of the acoustically transparent section transitions from circular to elliptical as the acoustically transparent section extends distally.

13. The ultrasound catheter for interventional treatment according to claim 9, wherein the cross-sectional profile of the acoustically transparent section is elliptical; and
the ratio of a minor axis to a major axis of the elliptical acoustically transparent section is 1:1.7 to 1:2.3, and/or the ratio of a minimum wall thickness to a maximum wall thickness of the elliptical acoustically transparent section is 1:4 to 1:6.

14. The ultrasound catheter for interventional treatment according to any one of claims 9 to 13, wherein the first core lumen and the second core lumen are both cylindrical cavities, and the core can move forwards or backwards relative to the first core lumen and the second core lumen; and the ultrasound transducer has an initial position substantially flush with the guidewire outlet, and the ultrasound transducer has a detection position when moving relative to the guidewire outlet.

15. The ultrasound catheter for interventional treatment according to any one of claims 9 to 13, wherein the rapid exchange section has a length L1, the distance from the distal end of the ultrasound catheter to the proximal end of the acoustically transparent section is (L1 + Lt), and the ratio of L1 to (L1 + Lt) is 1/5 to 1/7.

16. The ultrasound catheter for interventional treatment according to any one of claims 9 to 13, wherein the acoustically transparent section is provided with an arc-shaped profile at the guidewire outlet, and the radial dimension of the acoustically transparent section gradually tapers as the acoustically transparent section extends distally; and/or
the rapid exchange section is provided with an arc-shaped hole for allowing the directional guidewire to pass through in a bent state.

17. The ultrasound catheter for interventional treatment according to any one of claims 9 to 13, wherein the dual-lumen microcatheter comprises an inner polymer layer, an intermediate reinforcement layer, and an outer resin layer in sequence from inside to outside;
the inner polymer layer is made of a polymer material with a low coefficient of friction, and the inner polymer layer forms the first guide lumen and the first core lumen;
the intermediate reinforcement layer is formed by braiding or winding around or sheathing the inner polymer layer;
the outer resin layer is made of at least two materials with different hardnesses, and the hardness of the outer resin layer gradually decreases from the proximal end to the distal end;
the inner polymer layer, the intermediate reinforcement layer, and the outer resin layer are formed into the ultrasound catheter by the co-extrusion technology or heat shrink tubing technology.

18. An interventional treatment system, comprising a number of guidewires and an ultrasound catheter, wherein the ultrasound catheter is the ultrasound catheter for interventional treatment according to any one of claims 1-8 or claims 9-17;
the guidewires include an interventional guidewire extending out through the guidewire outlet and a directional guidewire passing through the rapid exchange section;
the directional guidewire is configured for directing the ultrasound catheter to a desired position, and the interventional guidewire is configured for delivering to a target position under the imaging guidance of the ultrasound catheter;
the guidewire can be switched between a status of extending out through the guidewire outlet and a status of passing through the rapid exchange section, such that the guidewire can be switched between the directional guidewire and the interventional guidewire.

19. The interventional treatment system according to claim 18, further comprising an interventional medical device, wherein the interventional medical device comprises at least one of a working guidewire, a balloon, a stent, a rotary milling device, and a suction device;
the interventional medical device is configured for delivering to the target position along the guidewire, or the interventional medical device is configured for extending out through the guidewire outlet and delivering to the target position directly under the imaging guidance of the ultrasound catheter.

20. The interventional treatment system according to claim 18, wherein the interventional treatment system is configured for interventional occlusion treatment;
the guidewires further include an interventional treatment guidewire; the distal end of the interventional treatment guidewire is arranged at the distal end of an occluded vascular section, the proximal end of the interventional treatment guidewire is arranged at the proximal end of the occluded vascular section, and a middle section of the interventional treatment guidewire bypasses the occluded vascular section under the imaging guidance of the ultrasound catheter through an interlayer between the intima and media of the occluded vascular section.

21. The interventional treatment system according to claim 18, wherein the ultrasound catheter further comprises a Y-shaped connection member and a connector;
the Y-shaped connection member comprises a third core lumen in communication with the proximal end of the first core lumen and a third guide lumen in communication with the proximal end of the first guide lumen; the interventional guidewire sequentially passes through the third guide lumen of the Y-shaped connection member, the first guide lumen of the dual-lumen microcatheter, and the second guide lumen of the acoustically transparent section and extends out through the guidewire outlet; the connector comprises an irrigation port and a core port, both in communication with the third core lumen.
